# EUROPEAN PATENT APPLICATION

(11) **EP 4 008 305 A1**
(43) Date of publication of application: **08.06.2022**
(21) Application number: 20212310.5
(22) Date of filing: 07.12.2020
(51) Int. Cl.: A61K 8/06, A61K 8/37, A61K 8/891, A61K 8/55, A61K 8/81, A61K 8/41, A61K 8/86, A61Q 19/00

(54) **OIL-IN-WATER COMPOSITION COMPRISING OIL DROPLETS WITH DIAMETER OF 0,1MM OR MORE**

(71) Applicant: Clariant International Ltd, 4132 Muttenz (CH)
(72) Inventor: LI, Jinglan, Tokyo, 113-0021 (JP); HAYASE, Mayu, Tokyo, 113-0021 (JP)
(74) Representative: Kampen, Daniela

(57) **Abstract**

The present invention relates to an oil-in-water (o/w) composition. The o/w composition comprises: (i) 100 parts by weight of water, (ii) 0.05 to 3 parts by weight of an oil, (iii) 0.1 to 10 parts by weight of an emulsifier, and (iv) 0.01 to 3 parts by weight of a thickner, wherein the o/w composition comprises oil droplets with diameter of 0.1 mm or more.

## Description

### FIELD OF INVENTION

The present invention relates to an oil-in-water (o/w) composition dispersing oil particles of 0.1 mm or larger and a method of manufacturing thereof.

### TECHNICAL BACKGROUND OF INVENTION

An o/w composition comprising large size of oil particles is required. JP2007-254405 discloses an o/w emulsified cosmetic composition comprising emulsified droplets having particle size of 100 to 500 nm, the composition comprises (A) oil agent that is liquid at room temperature, (B) hydrogenated phospholipid, (C) a nonionic surfactant having HLB of 9 to 16, (D) divalent glycols, and (E) water.

### BRIEF SUMMARY OF INVENTION

An objective is to provide an o/w composition comprising large size of oil droplets.

An aspect of the invention relates to an oil-in-water (o/w) composition comprising: (i) 100 parts by weight of water, (ii) 0.05 to 3 parts by weight of an oil, (iii) 0.1 to 10 parts by weight of an emulsifier, and (iv) 0.01 to 3 parts by weight of a thickner, wherein the o/w composition comprises oil droplets with diameter of 0.1 mm or more. Another aspect of the invention relates to a method of manufacturing the oil-in-water (o/w) composition of claim 1, the method comprises steps of: - preparing a water solution by mixing 100 parts by weight of water; 0.1 to 10 parts by weight of an emulsifier; and 0.01 to 3 parts by weight of a thickner, - preparing 0.05 to 3 parts by weight of an oil, - mixing the water solution and the oil to disperse the oil in the water solution, wherein the o/w composition comprises oil droplets with diameter of 0.1 mm or more.

An o/w composition comprising large size of oil droplets can be obtained by the present invention.

### BRIEF DESCRIPTION OF DRAWINGS

FIG. 1 (a) and FIG.1 (b) is a top-view and a side view of the o/w composition of Example 1 when measuring the oil droplets.
FIG. 2(a) and FIG. 2(b) is a top-view and a side view of the o/w composition of Example 2 when measuring the oil droplets.
FIG. 3(a) and FIG. 3(b) is a top-view and a side view of the o/w composition of Comparative Example 1 when measuring the oil droplets.

### DETAILED DESCRIPTION OF INVENTION

The o/w composition and the method of manufacturing thereof are explained.

The o/w composition comprises (i) 100 parts by weight of water, (ii) 0.05 to 3 parts by weight of an oil, (iii) 0.1 to 10 parts by weight of an emulsifier, and (iv) 0.01 to 3 parts by weight of a thickner.

### (i) Water

The water is a basic component of the o/w composition. There is no limitation on the water. The water can be ion-exchange water, distilled water, well water, tap water or a combination thereof in an embodiment. The water can be 80.0 weight percent (wt. %) or more in an embodiment, 85.0 wt. % or more in another embodiment, 89.5 wt. % or more in another embodiment, 94.0 wt. % or more in another embodiment, based on the weight of the o/w composition. The water is 99.8 wt. % or less in an embodiment, 98.5 wt. % or less in another embodiment, based on the weight of the o/w composition.

### (ii) Oil

The o/w composition comprises an oil.

Melting point of the oil is 40 °C or lower in an embodiment, 35 °C or lower in another embodiment, 30 °C or lower in another embodiment, 29 °C or lower in another embodiment. Melting point of the oil is 0 °C or higher in an embodiment, 5 °C or higher in another embodiment, 12 °C or higher in another embodiment, 18 °C or higher in another embodiment.

The oil comprises a polymerized structure in an embodiment. In other words, chemical structure of the oil comprises one or more of polymerized structure in another embodiment. The polymerized structure means a structure that comprises two or more monomers repeatedly bonded. The oil is a polymeric compound in an embodiment. The oil of polymeric compound comprises homopolymer, copolymer, terpolymer or a combination thereof.

The oil is selected from the group consisting of a hydrocarbon oil, a fatty acid oil, an alcohol oil, an ester oil, an ether oil, a silicone oil and a combination thereof in an embodiment; an ester oil, a silicone oil and a combination thereof in another embodiment; an ester oil in another embodiment; a silicone oil in another embodiment.

The ester oil comprises an estolide ester in another embodiment. The estolide ester is an ester of an estolide with an alcohol. The estolide is an ester of same or different hydroxy fatty acids or hydroxy unsaturated fatty acids in an embodiment. The estolide is a polymer of hydroxy fatty acids or hydroxy unsaturated fatty acids in another embodiment. The estolide is a polymer of hydroxy unsaturated fatty acids in another embodiment. The estolide is 2 to 11 equivalents of hydroxy unsaturated fatty acids in another embodiment, 3 to 9 equivalents of hydroxy unsaturated fatty acids in another embodiment, 4 to 8 equivalents of hydroxy unsaturated fatty acids in another embodiment. The hydroxy unsaturated fatty acid is ricinoleic acid in another embodiment.

The alcohol is a fatty alcohol in an embodiment. Carbon number of the fatty alcohol is 3 to 20 in an embodiment, 6 to 18 in another embodiment, 10 to 16 in another embodiment, 12 to 14 in another embodiment. The fatty alcohol is selected from the group consisting of butyl alcohol, amyl alcohol, enanthic alcohol, capryl alcohol, pelargonic alcohol, capric alcohol, undecyl alcohol, lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol and arachidyl alcohol and a combination thereof in another embodiment; capryl alcohol, capric alcohol, undecyl alcohol, lauryl alcohol, tridecyl alcohol, myristyl alcohol, pentadecyl alcohol, stearyl alcohol and a combination thereof in another embodiment; lauryl alcohol, myristyl alcohol and a combination thereof in another embodiment; a combination of lauryl alcohol and myristyl alcohol in another embodiment.

The estolide ester is an ester of polyricinoleate with lauryl alcohol and/or myristyl alcohol in another embodiment, an ester of 2 to 11 equivalents polyricinoleate with lauryl alcohol and/or myristyl alcohol in another embodiment, an ester of 2 to 11 equivalents polyricinoleate with lauryl alcohol and myristyl alcohol in another embodiment.

The estolide ester of a polyricinoleate with lauryl alcohol and myristyl alcohol is called Lauryl/myristyl polyricinoleate in INCI name. The lauryl/myristyl polyricinoleate is available as a product in the market, for example GENADVANCE^{®} HYDRA from Clariant.

The silicone oil is selected from the group consisting of a dimethyl silicone oil, an organomodified silicone oil and a combination thereof in another embodiment.

The dimethyl silicone oil is expressed with formula (1):

Me₃SiO-[SiMe₂O]ₓ-SiMe₃ (1)

wherein x is 2 or more, and Me is a methyl group in an embodiment.

In formula (1), x is 3 or more in another embodiment, 5 or more in another embodiment, 7 or more in another embodiment, 10 or more in another embodiment, 12 or more in another embodiment. In formula (1), x is 30 or less in an embodiment, 27 or less in another embodiment, 25 or less in another embodiment, 23 or less in another embodiment, 20 or less in another embodiment.

The organomodified silicone oil is a dimethyl silicone oil with methyl group of side chain is partially replaced with another organic group in an embodiment.

In another embodiment, the organomodified silicone oil is expressed with formula (2) or (3):

Me₃SiO-[Si(Me)(R)O]_{y}-SiMe₃ (2)

Me₃SiO-[[Si(Me₂)O]_{z}[Si(Me)(R)O]ᵥ]-SiMe₃ (3)

wherein R is independently an alkyl group, an alkenyl group, a phenyl group, an alkylaryl group or an arylalkyl group, and
y is an integer between 2 and 30,
z is an integer between 1 and 29,
v is an integer between 1 and 29,
z+v is between 3 and 30,
Me is a methyl group.

In formulae (2) or (3):
R is a linear, branched or cyclic organic group in another embodiment; R is a linear organic group in another embodiment;
R has 2 to 40 carbon atoms in an embodiment, 6 to 30 carbon atoms in another embodiment, 10 to 25 carbon atoms in another embodiment, 15 to 20 carbon atoms in another embodiment;
R is an alkyl group, an alkenyl group, a phenyl group, an alkylaryl group or an arylalkyl group in another embodiment; an alkyl group in another embodiment; an alkyl group selected from the group consisting of butyl, pentyl, hexyl, heptyl, octyl, nonyl, decyl, undecyl, dodecyl (lauryl), tridecyl, tetradecyl (myristyl), pentadecyl, hexadecyl (cetyl), octadecyl (stearyl), nonadecyl and icosyl in another embodiment; decyl, undecyl, dodecyl (lauryl), tridecyl, tetradecyl (myristyl), pentadecyl, hexadecyl (cetyl), octadecyl (stearyl) and nonadecyl in another embodiment; pentadecyl, hexadecyl (cetyl), octadecyl (stearyl) in another embodiment; octadecyl (stearyl) in another embodiment;
y is 1 or more in another embodiment, 2 or more in another embodiment, 3 or more in another embodiment, 5 or more in another embodiment, 20 or less in another embodiment, 15 or less in another embodiment, 10 or less in another embodiment, 7 or less in another embodiment;
z is 1 or more in another embodiment, 2 or more in another embodiment, 3 or more in another embodiment, 5 or more in another embodiment, 20 or less in another embodiment, 16 or less in another embodiment, 12 or less in another embodiment, 10 or less in another embodiment;
v is 1 or more in another embodiment, 2 or more in another embodiment, 20 or less in another embodiment, 16 or less in another embodiment, 12 or less in another embodiment, 10 or less in another embodiment;
z+v is 3 or more in another embodiment, 4 or more in another embodiment, 58 or less in another embodiment, 42 or less in another embodiment, 38 or less in another embodiment, 22 or less in another embodiment, 18 or less in another embodiment, 13 or less in another embodiment.

In another embodiment, the oil comprises an organomodified silicone oil of formula (4):

Me₃SiO-[[Si(Me₂)O]_{z}[Si(Me)(St)O]ᵥ]-SiMe₃ (4)

wherein z is between 1 and 29, v is an integer between 1 and 29, z+v is between 3 and 30, Me is methyl group and St is stearyl group. The oil is an organomodified silicone oil of formula (4) in another embodiment.

The dimethyl silicone with side chains of methyl group and stearyl group is also called stearyl dimethicone in INCI name. A stearyl dimethicone is available as a product in the market, for example SilCare^{®} Silicone 41M65 from Clariant.

The oil is selected from the group consisting of a dimethyl silicone with side chains of methyl group and stearyl group (stearyl dimethicone), an ester of polyricinoleate with lauryl alcohol and/or myristyl alcohol and a combination thereof in another embodiment. The oil comprises an ester of polyricinoleate with lauryl alcohol and/or myristyl alcohol in another embodiment. The oil is an ester of polyricinoleate with lauryl alcohol and/or myristyl alcohol in another embodiment.

The oil is a liquid phase at 0 to 50 °C in an embodiment, at 10 to 45 °C in another embodiment, at 15 to 36 °C in another embodiment, at 20 to 29 °C in another embodiment, at room temperature of about 25 °C in another embodiment.

The oil is 0.1 parts by weight or more in an embodiment, 0.15 parts by weight or more in another embodiment, 0.2 parts by weight or more in another embodiment, 0.25 parts by weight or more in another embodiment. The oil can be 3 parts by weight or less, 2.4 parts by weight or less in another embodiment, 1.9 parts by weight or less in another embodiment, 1.4 parts by weight or less in another embodiment, 0.9 parts by weight or less in another embodiment, 0.6 parts by weight or less in another embodiment, 0.4 parts by weight or less in another embodiment.

The oil is 0.08 wt. % or more in an embodiment, 0.12 wt. % or more in another embodiment, 0.16 wt. % or more in another embodiment, 0.2 wt. % or more in another embodiment, based on the weight of the o/w composition. The oil is 5 wt. % or less in an embodiment, 4.1 wt. % or less in another embodiment, 3.5 wt. % or less in another embodiment, 2.2 wt. % or less in another embodiment, 1.7 wt. % or less in another embodiment, 0.6 wt. % or less in another embodiment, based on the weight of the o/w composition.

### (iii) Emulsifier

The emulsifier is used to emulsify water and an oil. Hydrophilic-lipophilic balance (HLB) of the emulsifier is 2 or more in an embodiment, 3 or more in another embodiment, 4 or more in another embodiment, 5 or more in another embodiment, 6 or more in another embodiment, 7 or more in another embodiment, 8 or more in another embodiment, 9 or more in another embodiment, 10 or more in another embodiment, 11 or more in another embodiment, 12 or more in another embodiment. HLB of the emulsifier is 15 or less in an embodiment, 14 or less in another embodiment, 13 or less in another embodiment, 12 or less in another embodiment, 11 or less in another embodiment, 10 or less in another embodiment, 9 or less in another embodiment, 8 or less in another embodiment. The HLB refers to the value obtained by the Griffin's method. According to Griffin's method, determined as HLB=20*Mₕ/M where Mₕ is the molecular mass of the hydrophilic portion of the molecule, and M is the molecular mass of the whole molecule, giving a result on a scale of 0 to 20. The computation can be used to provide HLB value, for example a software program Molecular Modeling Pro from Norgwyn Montgomery Software, Inc. The emulsifier is selected from the group consisting of sulfonic acid compounds, sulfate ester compounds, phosphoric acid ester compounds, esters of polyalcohol and fatty acid and a combination thereof in another embodiment; phosphoric acid ester compounds, esters of polyalcohol and fatty acid and a combination thereof in another embodiment.

The sulfonic acid compound is selected from the group consising of dialkyl sulfosuccinates, alkyl sulfonic acid salts, olefin sulfonates, alkyl benzene sulfonates, and alkyl naphthalene sulfonates and a combination thereof in an embodiment.

The sulfate ester compound is selected from the group consising of alkyl sulfates, alkyl ether sulfates, sulfonated castor oils and a combination thereof in an embodiment.

The phosphoric acid ester compound is selected from the group consising of alkyl phosphates, polyoxyethylene alkyl ether phosphates, polyoxyethylene alkyl phenyl ether phosphates and a combination thereof in an embodiment. The phosphoric acid ester compound is selected from the group consisting of polyoxyethylene alkyl ether phosphates in another embodiment.

The alkyl group comprised in the sulfonic acid compound, sulfate ester compound, or phosphoric acid ester compound is independently selected from the group consisting of butyl, pentyl, hexyl, heptyl, octyl, nonyl, decyl, undecyl, dodecyl (lauryl), tridecyl, tetradecyl (myristyl), pentadecyl, hexadecyl (cetyl), octadecyl (stearyl), nonadecyl, and icosyl in another embodiment; decyl, undecyl, dodecyl (lauryl), tridecyl, tetradecyl (myristyl), pentadecyl, hexadecyl (cetyl), octadecyl (stearyl) and nonadecyl in another embodiment; dodecyl (lauryl), tridecyl, tetradecyl (myristyl), pentadecyl, hexadecyl (cetyl), octadecyl (stearyl) in another embodiment; dodecyl (lauryl), tridecyl, tetradecyl (myristyl) in another embodiment; dodecyl (lauryl) in another embodiment. The polyoxyethylene alkyl ether phosphate is polyoxyethylene lauryl ether phosphate in another embodiment.

Polyoxyethylene lauryl ether phosphate is trilaureth-4 phosphate in INCI name. Trilaureth-4 phosphate is available as a product in the market, for example Hostaphat^{®} KL 340D from Clariant.

The ester of polyalcohol and fatty acid is selected from the group consisting of glycerin fatty acid esters, polyglycerin fatty acid esters, sorbitan fatty acid esters, polysorbitan fatty acid esters, propylene glycol fatty acid esters, polypropylene glycol fatty acid esters, sucrose fatty acid esters, polysucrose fatty acid esters and a combination thereof in another embodiment.

The ester of polyalcohol and fatty acid is selected from the group consisting of glycerin fatty acid esters, polyglycerin fatty acid esters, sucrose fatty acid esters, polysucrose fatty acid esters and a combination thereof in another embodiment; glycerin fatty acid esters, polyglycerin fatty acid esters and a combination thereof in another embodiment.

The glycerin fatty acid ester is a compound comprising glycerin (HOCH₂CH(OH)CH₂OH) with one or two hydroxy group(s) esterified with fatty acid(s).

The polyglycerin fatty acid ester is a compound comprising polyglycerin with one or more hydroxy group(s) esterified with fatty acid(s). The polyglycerin is a linear, branched or cyclic polyglycerin in an embodiment. The polyglycerin is selected from the group consisting of diglycerin, triglycerin, tetraglycerin, pentaglycerin, hexaglycerin, heptaglycerin, octaglycerin, nonaglycerin, decaglycerin or mixture thereof in an embodiment.

The fatty acid of the glycerin fatty acid ester or the polyglycerin fatty acid ester is saturated fatty acids or unsaturated fsatty acids in an embodiment, a saturated fatty acid in another embodiment. The fatty acid of the glycerin fatty acid ester or the polyglycerin fatty acid ester is a linear or branched fatty acid in another embodiment, a branched fatty acid in another embodiment. The fatty acid of the glycerin fatty acid ester or the polyglycerin fatty acid ester has carbon number of 6 to 30 or a polymer thereof in another embodiment, 10 to 22 or a polymer thereof in another embodiment, 12 to 18 in another embodiment.

The fatty acid of the glycerin fatty acid ester or the polyglycerin fatty acid ester is selected from the group consisting of caproic acid, enanthic acid, capric acid, pelargonic acid, caproic acid, lauric acid, myristic acid, myristoleic acid, pentadecylic acid, palmitic acid, palmitoleic acid, sapienic acid, margaric acid, elaidic acid, vaccenic acid, alpha-linolenic acid, gamma-linolenic acid, pinolenic acid stearic acid, oleic acid, vaccenic acid, linoleic acid, linolenic acid, ricinoleate acid, arachidic acid, arachidonic acid, eicosenoic acid, eicosapentaenoic acid, gadoleic acid, behenic acid, docosahexaenoic acid, erucic acid, lignoceric acid, nervonic acid, cerotic acid, montanic acid, melissic acid, isocaproic acid, isoenanthic acid, isocapric acid, isopelargonic acid, isocaproic acid, isolauric acid, isomyristic acid, isopentadecanoic acid, isopalmitic acid, isomargaric acid, isostearic acid, isooleic acid, isovaccenic acid, isolinoleic acid, isolinolenic acid, isoarachidic acid, isoarachidonic acid, isobehenic acid, isolignoceric acid and isomontanic acid and a polymer thereof in another embodiment; lauric acid, myristic acid, myristoleic acid, pentadecanoic acid, palmitic acid, palmitoleic acid, sapienic acid, stearic acid, oleic acid, vaccenic acid, linoleic acid, linolenic acid, ricinoleate acid, isolauric acid, isomyristic acid, isopalmitic acid, isomargaric acid, isostearic acid, isooleic acid, isovaccenic acid, isolinoleic acid, isolinolenic acid, isoricinoleate acid, a polymer thereof in another embodiment; stearic acid, oleic acid, ricinoleic acid, isostearic acid isoricinoleate acid, a polymer thereof in another embodiment.

The glycerin fatty acid ester is a monoester, a diester or a combination thereof in another embodiment, a monoester in another embodiment.

The glycerin fatty acid ester is selected from the group consisting of glyceryl-caprylate, -isocaprylate, -caproate, -isocaproate, -stearate, -oleate, -isostearate, - caprylate, -ricinoleate, -isocaprylate, -laurate, -isolaurate, -myristate, -isomyristate, - palmitate, -isopalmitate -polycaprylate, -polyisocaprylate, -polycaproate, - polyisocaproate, -polystearate, -polyisostearate, -polycaprylate, -polyisocaprylate, - polyricinoleate, -polyisoricinoleate, -polylaurate, -polyisolaurate, -polymyristate, - polyisomyristate, -polypalmitate, -polyisopalmitate, and a combination thereof in another embodiment; glyceryl-stearate, -oleate, -isostearate, -ricinoleate, -laurate, - isolaurate, -myristate, -isomyristate, -palmitate, -isopalmitate, -polystearate, - polyisostearate, -polyricinoleate, -polyisoricinoleate, -polylaurate, -polyisolaurate and a combination thereof in another embodiment; glyceryl-stearate, -oleate, - polyricinoleate, and a combination thereof in another embodiment.

The polyglycerin fatty acid ester is a monoester, a diester, a triester, a tetraester, a pentaester, a hexaester, heptaester, octaester or a combination thereof in another embodiment; monoester, diester, or a combination thereof in another embodiment; a mixture of monoester and diester in another embodiment.

The polyglycerin fatty acid ester is selected from the group consisting of polyglyceryl-caprylate, -isocaprylate, -caproate, -isocaproate, -stearate, -isostearate, -caprylate, - isocaprylate, -laurate, -isolaurate, -myristate, -isomyristate, -palmitate, -isopalmitate, - ricinoleate, -isoricinoleate, -polycaprylate, -polyisocaprylate, -polycaproate, - polyisocaproate, -polystearate, -polyisostearate, -polycaprylate, -polyisocaprylate, - polylaurate, -polyisolaurate, -polymyristate, -polyisomyristate, -polyisopalmitate, - polyricinoleate and a combination thereof in another embodiment; polyglyceryl-stearate, -isostearate, -polystearate, -polyisostearate, -polyricinoleate and a combination thereof in another embodiment.

The polyglycerin fatty acid ester is selected from the group consisting of a diglyceryl-stearate, -distearate, -isostearate, -diisostearate and a combination thereof in another embodiment; diglyceryl-isostearate, diglyceryl-diisostearate and a combination thereof in another embodiment. The polyglycerin fatty acid ester is a combination of diglyceryl isostearate and diglyceryl polyisostearate in another embodiment, a combination of diglyceryl isostearate and diglyceryl diisostearate in another embodiment.

The emulsifier is selected from the group consisting of polyoxyethylene alkyl ether phosphates, glyceryl stearate, glyceryl oleate, polyglyceryl stearate, polyglyceryl isostearate, polyglyceryl diisostearate, polyglyceryl polyricinoleate and a combination thereof in another embodiment; polyoxyethylene lauryl ether phosphate, glyceryl stearate, glyceryl oleate, diglyceryl isostearate and diglyceryl diisostearate, triglyceryl polyricinoleate and a combination thereof in another embodiment.

Glyceryl stearate is available as a product in the market, for example Plantasens^{®} Emulsifier HP30 from Clariant.

The mixture of diglyceryl isostearate and diglyceryl diisostearate is polyglyceryl-2 sesquiisostearate in INCI name. Polyglyceryl-2 sesquiisostearate is available as a product in the market, for example Plantasens^{®} Emulsifier DGI from Clariant.

The mixture of glyceryl oleate and polyglyceryl-polyricinoleate is available as a product in the market, for example Plantasens^{®} Emulsifier CP5.

The emulsifier is 0.2 parts by weight or more in an embodiment, 0.3 parts by weight or more in another embodiment, 0.4 parts by weight or more in another embodiment, 0.5 parts by weight or more in another embodiment, 0.8 parts by weight or more in another embodiment, 0.9 parts by weight or more in another embodiment, 1.0 parts by weight or more in another embodiment. The emulsifier is 9.5 parts by weight or less in an embodiment, 8.1 parts by weight or less in another embodiment, 7.4 parts by weight or less in another embodiment, 6.5 parts by weight or less in another embodiment, 5.6 parts by weight or less in another embodiment, 4.5 parts by weight or less in another embodiment, 3.4 parts by weight or less in another embodiment, 2.9 parts by weight or less in another embodiment, 1.9 parts by weight or less in another embodiment, 1.2 parts by weight or less in another embodiment.

The emulsifier is 0.09 wt. % or more in an embodiment, 0.25 wt. % or more in another embodiment, 0.35 wt. % or more in another embodiment, 0.5 wt. % or more in another embodiment, 0.6 wt. % or more in another embodiment, 0.8 wt. % or more in another embodiment, based on the weight of the o/w composition. The emulsifier is 10 wt. % or less in an embodiment, 8.4 wt. % or less in another embodiment, 6.5 wt. % or less in another embodiment, 5.8 wt. % or less in another embodiment, 4.5 wt. % or less in another embodiment, 3.2 wt. % or less in another embodiment, 2.1 wt. % or less in another embodiment, based on the weight of the o/w composition. Another emulsifier with HLB of less than 4 or more than 15 can be further added to the o/w composition in an embodiment. The o/w composition further comprises another emulsifier with HLB of more than 14 in another embodiment. The o/w composition further comprises another emulsifier with HLB of less than 3 in another embodiment.

### (iv) Thickner

A thickner increases viscosity of an o/w composition. Any thickner is suitable for the o/w composition. The thickner can be chosen in view of desired viscosity of the o/w composition.

The thickner is a water soluble in an embodiment.

The thickner is selected from the group consisting of natural polymers, cellulose-based polymers, alginate-based polymers, vinyl polymers, acrylic polymers and a combination thereof in another eimbodiment.

The natural polymer is selected from the group consisting of arabian gum, tragacanto gum, galactan, guagam, carob gum, karaya gum, carrageenan, pectin, canten, quince seed, algae colloid, starch, glycyrrhizinic acid, xanthan gum, dextran, succinoglucan, burran, collagen, casein, albumin, gelatin, hyaluronic acid and a combination thereof in an embodiment.

The cellulose-based polymer is selected from the group methylcellulose, ethylcellulose, methylhydroxypropylcellulose, hydroxyethylcellulose, sodium cellulose sulfate, hydroxypropylcellulose, carboxymethylcellulose, sodium carboxymethylcellulose, crystalline cellulose, cellulose powder, O-[2-hydroxy-3-(trimethylammonio) propyl] hydroxyethyl cellulose and a combination thereof in an embodiment.

The alginate-based polymer is selected from the group consisting of sodium alginate, propylene glycol alginate and a combination thereof in an embodiment.

The vinyl polymer is selected from the group consisting of polyvinyl alcohol, polyvinyl methyl ether, polyvinylpyrrolidone, carboxyvinyl polymer and a combination thereof in an embodiment.

The acrylic polymer is selected from the group consisting of poly(acrylic acid), sodium polyacrylate, polyethyl acrylate, polyacrylamide, copolymer of acrylic acid and alkyl methacrylate, crosspolymer of dimethylacrylamide and acryloyldimethyltaurine salt, copolymer of 2-acrylamide-2-methylpropanesulfonic acid and vinylpyrrolidone, copolymer of hydroxyethyl acrylate and acryloyl dimethyl taurine salt, copolymer of acrylate and acryloyl dimethyl taurine salt, copolymer of ammonium acryloyldimethyltaurine and vinyl pyrrolidone (VP), crosspolymer of ammonium acryloyldimethyltaurine and behenes-25 methacrylate and a combination thereof in an embodiment; poly(acrylic acid), crosspolymer of dimethylacrylamide and acryloyldimethyltaurine salt, copolymer of 2-acrylamide-2-methylpropanesulfonic acid and vinylpyrrolidone, copolymer of hydroxyethyl acrylate and acryloyl dimethyl taurine salt, copolymer of acrylate and acryloyl dimethyl taurine salt, copolymer of ammonium acryloyldimethyltaurine ammonium and vinyl pyrrolidone (VP), crosspolymer of ammonium acryloyldimethyltaurine and behenes-25 methacrylate and a combination thereof in another embodiment; poly(acrylic acid), copolymer of ammonium acryloyldimethyltaurine ammonium and vinyl pyrrolidone (VP), crosspolymer of ammonium acryloyldimethyltaurine and behenes-25 methacrylate and a combination thereof in another embodiment. The acrylic polymer is crosspolymer of ammonium acryloyldimethyltaurine and behenes-25 methacrylate in another embodiment.

The thickner is acrylic polymer in another embodiment. The acrylic polymer is selected from the group consisting of copolymer of ammonium acryloyldimethyltaurine and vinyl pyrrolidone (VP), crosspolymer of ammonium acryloyldimethyltaurine and behenes-25 methacrylate and a combination thereof in another embodiment. The acrylic polymer is a crosspolymer of ammonium acryloyldimethyltaurine and behenes-25 methacrylate in another embodiment.

The crosspolymer of ammonium acryloyldimethyltaurine and behenes-25 methacrylate is available as a product in the market, for example Aristoflex^{®} BLV from Clariant.

The thickner is 0.05 to 3 parts by weight in an embodiment.

The thickner is 0.05 parts by weight or more in an embodiment, 0.1 parts by weight or more in another embodiment, 0.15 parts by weight or more in another embodiment, 0.2 parts by weight or more in another embodiment, 0.25 parts by weight or more in another embodiment. The thickner is 2.8 parts by weight or less in an embodiment, 2.4 parts by weight or less in another embodiment, 1.9 parts by weight or less in another embodiment, 1.4 parts by weight or less in another embodiment, 0.9 parts by weight or less in another embodiment, 0.6 parts by weight or less in another embodiment, 0.5 parts by weight or less in another embodiment.

The thickner is 0.04 wt. % or more in an embodiment, 0.1 wt. % or more in another embodiment, 0.18 wt. % or more in another embodiment, 0.25 wt. % or more in another embodiment, based on the weight of the o/w composition. The thickner is 5 wt. % or less in an embodiment, 4.1 wt. % or less in another embodiment, 3.5 wt. % or less in another embodiment, 2.8 wt. % or less in another embodiment, 1.9 wt. % or less in another embodiment, 1.5 wt. % or less in another embodiment, 1.0 wt. % or less in another embodiment, 0.7 wt. % or less in another embodiment, based on the weight of the o/w composition.

### (v) Additive

The o/w composition may comprise an additive such as a preservative, a scent, a colorant, a moisturizer, an emollient, a whitening agent, an anti-aging agent, or a gloss agent.

The additive is 0.05 parts by weight or more in an embodiment, 0.1 parts by weight or more in another embodiment, 0.25 parts by weight or more in another embodiment, 0.3 parts by weight or more in another embodiment, 0.45 parts by weight or more in another embodiment, 0.5 parts by weight or more in another embodiment. The additive is 5 parts by weight or less in an embodiment, 4.1 parts by weight or less in another embodiment, 3.5 parts by weight or less in another embodiment, 2.6 parts by weight or less in another embodiment, 1.9 parts by weight or less in another embodiment, 1.5 parts by weight or less in another embodiment, 1.0 parts by weight or less in another embodiment.

The additive is 0.01 wt. % or more in an embodiment, 0.26 wt. % or more in another embodiment, 0.5 wt. % or more in another embodiment, 1.0 wt. % or more in another embodiment, 2.2 wt. % or more in another embodiment, based on the weight of the o/w composition. The additive is 5 wt. % or less in an embodiment, 4.4 wt. % or less in another embodiment, 3.8 wt. % or less in another embodiment, 2.9 wt. % or less in another embodiment, 2.0 wt. % or less in another embodiment, 1.7 wt. % or less in another embodiment, 1.2 wt. % or less in another embodiment, based on the weight of the o/w composition.

### O/W composition

The o/w composition comprises oil droplets with diameter of 0.1 mm or more. The diameter of droplets can be 0.2 mm or more in another embodiment, 0.3 mm or more in another embodiment, 0.5 mm or more in another embodiment, 0.7 mm or more in another embodiment, 0.8 mm or more in another embodiment. The o/w composition comprises oil droplets with diameter of 3.0 mm or less in another embodiment, 2.5 mm or less in another embodiment, 2.1 mm or less in another embodiment, 1.8 mm or less in another embodiment, 1.6 mm or less in another embodiment, 1.2 mm or less in another embodiment. The diameter of droplets can be visually measured with a ruler. A loupe or an optical microscope is available. The diameter of droplets can be determined by an average of 100 oil droplets randomly selected.

There is no restriction on viscosity of the o/w composition. The viscosity of the o/w composition at 25 °C is at least higher than that of water in an embodiment. The viscosity of the o/w composition at 25 °C is 1 mPa·s or more in an embodiment, 100 mPa·s or more in another embodiment, 200 mPa·s or more in another embodiment, 400 mPa·s or more in another embodiment, 600 mPa·s or more in another embodiment, 1 Pa·s or more in another embodiment, 2.5 Pa·s or more in another embodiment, 3.8 Pa·s or more in another embodiment, 4.5 Pa·s or more in another embodiment, 5.2 Pa·s or more in another embodiment, 7 Pa·s or more in another embodiment. The viscosity of the o/w composition at 25 °C is 10 Pa·s or less in an embodiment, 9.2 Pa·s or less in another embodiment, 9.0 Pa·s or less in another embodiment, 7.0 Pa·s or less in another embodiment, 5.0 Pa·s or less in another embodiment, 4.2 Pa·s or less in another embodiment, 3.3 Pa·s or less in another embodiment, 2.3 Pa·s or less in another embodiment, 1500 mPa·s or less in another embodiment, 900 mPa·s or less in another embodiment.

Viscosity is measured with a digital viscometer (BROOKFIELD DVE, model RV) with a spindle #03 at 12 rpm, at about 25° C for 5 minutes.

There is no restriction on pH of the o/w composition. The pH of the o/w composition is 5 or higher in an embodiment, 6 or higher in another embodiment. The pH of the o/w composition is 8 or lower in an embodiment, 7 or lower in another embodiment. There is no restriction on colour of the o/w composition. The colour of the water phase is white or transparent unless a colorant is added in an embodiment. The colour of the oil droplets is white or transparent unless a colorant is added in an embodiment. The viscosity, pH and colour can be adjusted depending on how to use the o/w composition.

The o/w composition can be used for a medical product or a cosmetic product in an embodiment. A medical product or a cosmetic product comprises the o/w composition described above in an embodiment. The cosmetic product is a skin care product or a hair care product in an embodiment. The medical product is selected from the group consisting of an anti-inflammatory drug and a hair grower in another embodiment. The cosmetic product is selected from the group consisting of a skin lotion, moisturizing agent, and a hair lotion in another embodiment.

### Manufacturing method of o/w composition

A method of manufacturing the o/w composition comprises steps of preparing a water solution by mixing 100 parts by weight of water, 0.1 to 10 parts by weight of an emulsifier, and 0.01 to 3 parts by weight of a thickner; preparing 0.05 to 3 parts by weight of an oil; mixing the water solution and the oil to disperse the oil in the water solution.

The water solution is prepared by mixing water, an emulsifier and a thickner in a water-bath in an embodiment. Temperature of the water-bath is 30 to 90 °C in an embodiment, 35 to 78 °C in another embodiment, 42 to 69 °C in another embodiment, 53 to 65 °C in another embodiment. In an embodiment, water and an emulsifier are mixed and then a thickner is added to the mixture of water and the emulsifier. The o/w composition is obtained by mixing the water solution and an oil in the water-bath in an embodiment. A mixer with a blade can be used to mix the oil and the water solution in an embodiment. A mixer blade rotates at 100 to 800 rpm in an embodiment, 250 to 710 rpm in another embodiment, 340 to 630 rpm in another embodiment, 420 to 580 rpm in another embodiment. Mixing time is 1 to 30 minutes in an embodiment, 2.5 to 18 minutes in another embodiment, 3.5 to 10 minutes in another embodiment. An additive can be added to the o/w composition after cooling down to room temperature in an embodiment.

### EXAMPLE

### Example 1, 2, Comparative Example 1, 2

An o/w composition was prepared with following components. The components are expressed with INCI (International Nomenclature for Cosmetic Ingredients Name).

### (i) Water: ion exchanged water

### (ii) Oil

- Lauryl/Myristyl Polyricinoleate (Genadvance^{®} Hydra from Clariant)
- Stearyl dimethicone (SilCare^{®} Silicone 41M65 from Clariant)
- Quaternium-98 (Genadvance^{®} Repair from Clariant): A complex ammonium salt obtained by reacting ethoxylated laurylpropylene diamine with lauric acid and then quaternizing with dimethyl sulfate.
- Hydrogenated ethylhexyl olivate and hydrogenated olive oil unsaponifiables (Plantasens^{®} Olive LD from Clariant): A mixture of hydrogenated ethylhexyl olivate and hydrogenated olive oil unsaponifiables.

### (iii) Emulsifier

- Trilaureth-4 phosphate (HLB 14, Hostaphat^{®} KL 340D from Clariant)

### (iv) Thickner

- Ammonium acryloyldimethyltaurate/beheneth-25 methacrylate crosspolymer (Aristoflex^{®} BLV from Clariant)

### (v) Additive

- Capryloyl/caproyl anhydro methyl glucamide (Velsan^{®} Flex from Clariant)
- Phenoxyethanol

Water and an emulsifier were mixed with a stirring rod in a water-bath of 60 °C, followed by adding a thickner to further mixing until the emulsifier and the thickner were dissolved in water. An oil was added to the water solution in the water-bath and mixed by using a mixer (Tornado Standard, SM-102, AS-ONE Corporation) with a three-blade. The mixing was gently carried out for about 5 minutes at about 500 rpm to get the oil dispersed in the water solution with little entry of air bubbles into the mixture. The o/w composition was obtained by cooling down the mixture to room temperature (about 25 °C). Then the additives were added to the mixture and mixed for 5 minutes at 500 rpm. Viscosity of the o/w composition was between 550 to 8900 mPa·s at 25 °C. The components and their amount of the o/w composition in each example are shown in Table 1.

### Measurement of oil droplet

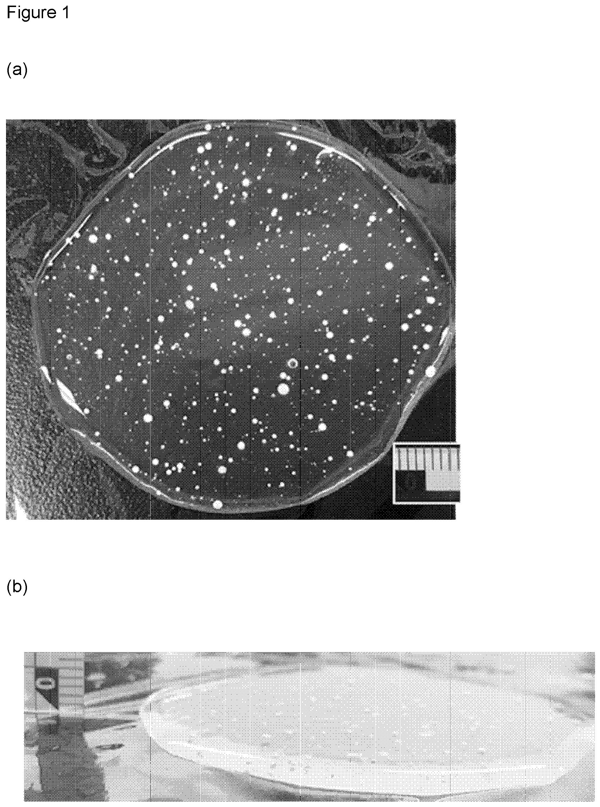

After having left the o/w composition produced above to stand for one month, the oil droplet dispersed in the o/w composition was measured in size. The o/w composition of 5 ml was poured onto a clear film spread on a flat black board as shown in FIG. 1(a). The diameter of droplets was visually measured with a ruler as determined by an average of 100 oil droplets randomly selected.

### Result

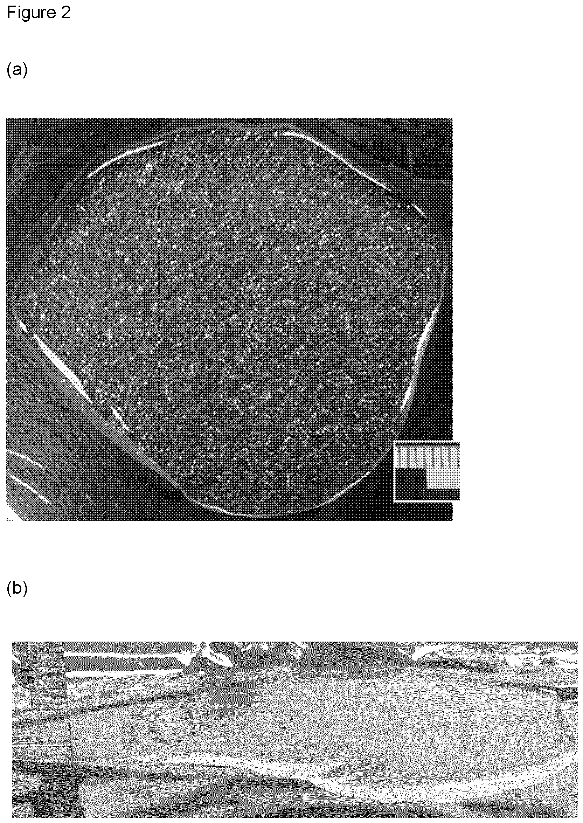

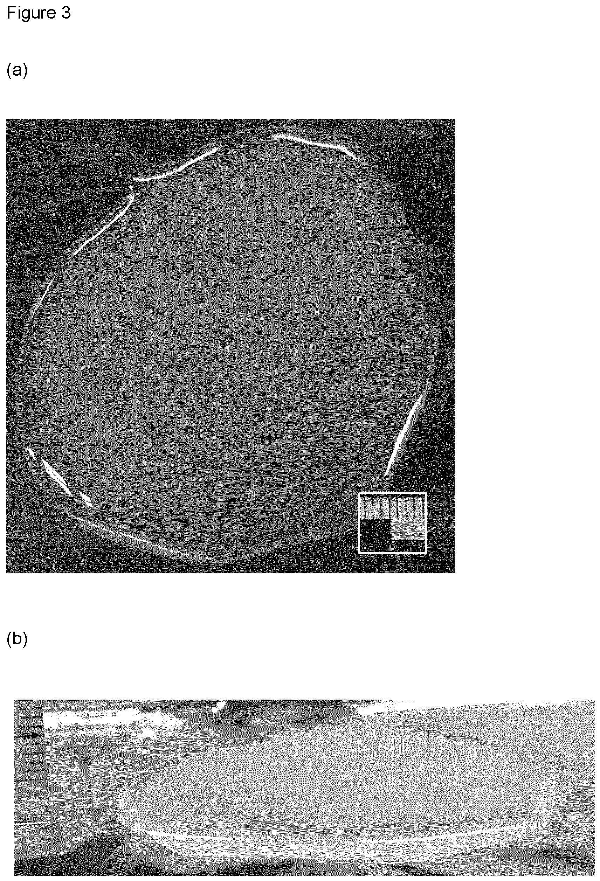

The o/w composition of Example 1 is shown in FIG. 1(a) of top view and FIG. 1(b) of side view, Example 2 is shown in FIG. 2(a) of top view and FIG. 2(b) of side view. The oil droplet diameter was 1.0 mm and 0.3 respectively when the o/w composition comprised lauryl/myristyl polyricinoleate in Example 1 and stearyl dimethicone in Example 2. The oil droplet diameter was too small to measure when the o/w composition comprised an oil of quaternium-98 or hydrogenated ethylhexyl olivate, hydrogenated olive oil unsaponifiables in Comparative (Com.) Example 1 and 2. The o/w composition of Com. Example 1 is shown in FIG. 3(a) of top view and FIG. 3(b) of side view where the oil droplets was so small to not measure (NM).

**Table 1 (parts by weight)**

| Component | INCI | Example 1 | Example 2 | Com. Example 1 | Com. Example 2 |
|---|---|---|---|---|---|
| Water | | 100 | 100 | 100 | 100 |
| Oil | Lauryl/myristyl polyricinoleate¹⁾ | 0.3 | | | |
| | Stearyl dimethicone²⁾ | | 0.3 | | |
| | Quaternium-98³⁾ | | | 0.3 | |
| | Hydrogenated ethylhexyl olivate, hydrogenated olive oil unsaponifiables⁴⁾ | | | | 0.3 |
| Emulsifier | Trilaureth-4 phosphate⁵⁾ | 1.0 | 1.0 | 1.0 | 1.0 |
| Thickner | Ammonium acryloyldimethyl-taurate/beheneth-25 methacrylate crosspolymer⁶⁾ | 0.3 | 0.3 | 0.3 | 0.3 |
| Additive | Capryloyl/caproyl anhydro methyl glucamide⁷⁾ | 0.3 | 0.3 | 0.3 | 0.3 |
| | Phenoxyethanol | 0.5 | 0.5 | 0.5 | 0.5 |
| Oil droplet diameter | | 1.0 mm | 0.3 mm | NM* | NM* |

| | | | | | |
|---|---|---|---|---|---|
| ¹⁾ Genadvance^{®} Hydra, ²⁾ SilCare^{®} Silicone 41M65, ³⁾ Genadvance^{®} Repair, ⁴⁾ Plantasens^{®} Olive LD, ⁵⁾ Hostaphat^{®} KL 340D, ⁶⁾ Aristoflex^{®} BLV, ⁷⁾ Velsan^{®} Flex, * Not Measured | | | | | |

### Example 3, Comparative Example 3

Next the emulsifier was examined. O/w compositions were prepared in the same manner of Example 1 except for replacing the emulsifier with one of the following emulsifiers as shown in Table 2.

### (iii) Emulsifier

- Glyceryl stearate (60 wt. % of glyceryl stearate, HLB 8, Plantasens^{®} Emulsifier HP30 from Clariant)
- Polyglyceryl-2 sesquiisostearate (HLB 5, Plantasens^{®} Emulsifier DGI from Clariant)
- PPG-5-Ceteth-20 (HLB 16, Sensotain^{™} EP6205 from Clariant)

The oil droplet diameter measured by the method described above is shown in Table 2. The oil droplet diameter was 1.0 mm, 0.3 mm and 0.4 mm when the o/w composition comprised an emulsifier of glyceryl stearate in Example 3. The oil droplet diameter was too small to measure when the o/w composition comprised an emulsifier of PPG-5-Ceteth-20 in Comparative Example 3.

**Table 2 (parts by weight)**

| Component | INCI | Example 3 | Com. Example 3 |
|---|---|---|---|
| Water | | 100 | 100 |
| Oil | Lauryl/Myristyl polyricinoleate¹⁾ | 0.3 | 0.3 |
| Emulsifier | Glyceryl stearate⁸⁾ | 1.0 | |
| | PPG-5-Ceteth-20⁹⁾ | | 1.0 |
| Thickner | Ammonium acryloyldimethyl-taurate/beheneth-25 methacrylate crosspolymer⁶⁾ | 0.3 | 0.3 |
| Additive | Capryloyl/caproyl anhydro methyl glucamide⁷⁾ | 0.3 | 0.3 |
| | Phenoxyethanol | 0.5 | 0.5 |
| Oil droplet diameter | | 1.0 mm | NM* |

| | | | |
|---|---|---|---|
| ¹⁾ Genadvance^{®} Hydra, ⁸⁾ Plantasens^{®} Emulsifier HP30 (60% of glyceryl stearate), ⁹⁾ Sensotain EP6205, ⁶⁾ Aristoflex^{®} BLV, ⁷⁾ Velsan^{®} Flex, * Not Measured | | | |

### Example 4, 5, 6, Comparative Example 4

Next, the amount of emulsifier, oil and thickner were examined. An o/w composition was prepared in the same manner of Example 1 except for changing the amount of the emulsifier, the oil or the thickner as shown in Table 3. The oil droplet diameter measured by the method described above is shown in Table 3. The oil droplet diameter was 1.0 mm in Example 4 to 6. The oil droplet size was too small to measure when comprising no thickner in Com. Example 4.

**Table 3 (parts by weight)**

| Component | INCI | Example 4 | Example 5 | Example 6 | Com. Example 4 |
|---|---|---|---|---|---|
| Water | | 100 | 100 | 100 | 100 |
| Oil | Lauryl/myristyl polyricinoleate¹⁾ | 0.3 | 0.2 | 0.3 | 0.3 |
| Emulsifier | Trilaureth-4 phosphate⁵⁾ | 5.0 | 1.0 | 1.0 | 1.0 |
| Thickner | Ammonium acryloyldimethyl-taurate/beheneth-25 methacrylate crosspolymer⁶⁾ | 0.3 | 0.3 | 0.2 | 0 |
| Additive | Capryloyl/caproyl anhydro methyl glucamide⁷⁾ | 0.3 | 0.3 | 0.3 | 0.3 |
| | Phenoxyethanol | 0.5 | 0.5 | 0.5 | 0.5 |
| Oil droplet size | | 1.0 mm | 1.0 mm | 1.0 mm | NM* |

| | | | | | |
|---|---|---|---|---|---|
| ¹⁾ GENADVANCE^{®} HYDRA, ⁵⁾ Hostaphat^{®} KL 340D, ⁶⁾ Aristoflex^{®} BLV, ⁷⁾ Velsan^{®} Flex * Not Measured | | | | | |

## Claims

1. An oil-in-water (o/w) composition comprising:
(i) 100 parts by weight of water,
(ii) 0.05 to 3 parts by weight of an oil,
(iii) 0.1 to 10 parts by weight of an emulsifier, and
(iv) 0.01 to 3 parts by weight of a thickner,
wherein the o/w composition comprises oil droplets with diameter of 0.1 mm or more.

2. The o/w composition of claim 1, wherein the oil comprises a polymerized structure.

3. The o/w composition of claim 1, wherein the oil is selected from the group consisting of a hydrocarbon oil, a fatty acid oil, an alcohol oil, an ester oil, an ether oil, a silicone oil and a combination thereof.

4. The o/w composition of any of claims 1 to 3, wherein the oil is selected from the group consisting of an ester oil, a silicone oil and a combination thereof.

5. The o/w composition of claim 3 or 4, wherein the ester oil comprises an estolide ester, the estolide ester is an ester of same or different hydroxy fatty acids or hydroxy unsaturated fatty acids.

6. The o/w composition of any of claims 1 to 4, wherein the oil is selected from the group consisting of a dimethyl silicone with side chains of methyl group and stearyl group (stearyl dimethicone), an ester of polyricinoleate with lauryl alcohol and/or myristyl alcohol and a combination thereof.

7. The o/w composition of any of claims 1 to 6, wherein hydrophilic-lipophilic balance (HLB) of the emulsifier is 2 to 15.

8. The o/w composition of any of claims 1 to 7, wherein the emulsifier is selected from the group consisting of sulfonic acid compounds, sulfate ester compounds, phosphoric acid ester compounds, esters of polyalcohol and fatty acid and a combination thereof.

9. The o/w composition of any of claims 1 to 8, wherein the emulsifier is selected from the group consisting of polyoxyethylene alkyl ether phosphates, glyceryl stearate, glyceryl oleate, polyglyceryl stearate, polyglyceryl isostearate, polyglyceryl diisostearate, polyglyceryl polyricinoleate and a combination thereof.

10. The o/w composition of any of claims 1 to 9, wherein the emulsifier is selected from the group consisting of polyoxyethylene lauryl ether phosphate (trilaureth-4 phosphate), glyceryl stearate, a mixture of diglyceryl isostearate and diglyceryl diisostearate (polyglyceryl-2 sesquiisostearate) and a combination thereof.

11. The o/w composition of any of claims 1 to 10, wherein viscosity of the o/w composition at 25 °C is 1 mPa·s to 10 Pa·s.

12. The o/w composition of any of claims 1 to 11, wherein the o/w composition comprises oil droplets with diameter of 3.0 mm or less.

13. A method of manufacturing the oil-in-water (o/w) composition of claim 1, the method comprises steps of:
- preparing a water solution by mixing 100 parts by weight of water; 0.1 to 10 parts by weight of an emulsifier; and 0.01 to 3 parts by weight of a thickner,
- preparing 0.05 to 3 parts by weight of an oil,
- mixing the water solution and the oil to disperse the oil in the water solution,
wherein the o/w composition comprises oil droplets with diameter of 0.1 mm or more.
